# EUROPEAN PATENT APPLICATION

(11) **EP 3 260 105 A1**
(43) Date of publication of application: **27.12.2017**
(21) Application number: 15882566.1
(22) Date of filing: 17.02.2015
(51) Int. Cl.: A61H 19/00, A61F 5/453, A61F 6/04

(54) **PRESSURE REDUCING DEVICE FOR EJACULATION PROMOTING APPARATUS**

(71) Applicant: Tenga Co., Ltd., Tokyo 108-0073 (JP)
(72) Inventor: MATSUURA, Tsutomu, Tokyo 108-0073 (JP)
(74) Representative: von Hirschhausen, Helge
(86) International application number: PCT/JP2015/054347
(87) International publication number: WO 2016/132462

(57) **Abstract**

Provided is a decompression device 50 by which varied stimulus and tightening force to a penis by an appropriate pressure can be obtained by adjusting increase/decrease in a pressure in a container 2 as a pressure source that causes the stimulus and the tightening force. There is provided the decompression device 50 being mounted to the ejaculation promotion device 1 including an ejaculation promotion apparatus 10 and a container 2, and being configured to decompress the container 2, the ejaculation promotion apparatus 10 being made of a gel-like resin and including an insertion empty space 15 and a valve section 10a that enables communication between an inside and an outside of the insertion empty space 15, the container 2 accommodating the ejaculation promotion apparatus 10, and the decompression device 50 includes: a negative pressure introducing section 55 that communicates with an air vent hole 3b provided in the container; a negative pressure generating section 53 that generates a negative pressure to be introduced into the container 2 through the negative pressure introducing section 55 and the air vent hole 33; and a negative pressure adjusting section 60 that adjusts the negative pressure supplied to the container 2 through the negative pressure introducing section 55.

## Description

The present invention relates to a decompression device that can further increase use efficiency of ejaculation promotion devices that have been conventionally used on the basis of a demand for medical study and treatment, and for such social demands as avoidance of sexual crimes, prostitution, and spreading of sexually transmitted diseases.

Various ejaculation promotion devices (sperm sampling devices) for sampling sperms of males have been proposed for needs of medical study and treatment. For example, the ejaculation promotion devices are used for medical needs such as an examination of a sexual function of the husband from sperms sampled to determine the cause of infertility of a couple, treatment of sexual functional disorder, and securing and storing the sperms for artificial insemination. Further, to meet various social needs such as prevention of sexual crimes by fulfilling personal sexual needs, avoidance of prostitution, and a decrease in the number of sexual disease infected patients, simplified ejaculation promotion devices are known, which have been conventionally available at a low price and do not raise hygiene and health problems because they are disposable or washable.

For example, Japanese Patent Application No. 2006-334176 A discloses an ejaculation promotion device that includes a tubular container made of relatively hard plastic with one end in a longitudinal direction open, and a core member made of a gel-like resin accommodated in the container and including an insertion empty space extending inward from an insertion hole in the one end in the longitudinal direction.

In this ejaculation promotion device, when a penis is inserted into the insertion empty space of the core member and expands the core member, an internal pressure in a space formed between the core member and the container cannot be decompressed to a predetermined value or less, and thus there is a limit on an increase in tightening force to the penis.

That is, there are individual differences in strength of a stimulus that promotes ejaculation of a user, and a weak stimulus is sufficient for some and a strong stimulus is necessary for others to get a desired effect. To obtain the strong stimulus, an increase in adhesion force (absorption force) of the core member to a penis front end section (glans) is effective. That is, to increase the stimulus to be added to the penis front end section when the penis rubs in a state of being inserted in the insertion empty space, decompression of the internal pressure in the space formed between the core member and the container to increase the adhesion force (absorption force) between the penis front end section and the core main body is effective. However, there is a limit on such decompression with the configuration of the Japanese Patent Application No. 2006-334176 A.

The present invention has been made in view of the foregoing, and an objective is to provide a decompression device for an ejaculation promotion device by which varied stimulus and tightening force to a penis by an appropriate pressure can be obtained by adjusting increase/decrease in a pressure in a container as a pressure source that causes the stimulus and the tightening force to the penis.

That is, by decompressing the container and removing the air in the container, the tightening force to the penis can be adjusted according to the needs of a user.

In order to solve the above issue, there is provided a decompression device for an ejaculation promotion device, the decompression device being to be mounted to the ejaculation promotion device including an ejaculation promotion apparatus and a container, and being configured to decompress the container, the ejaculation promotion apparatus being made of a gel-like resin and including an insertion empty space extending inward from an insertion hole and a valve section that enables communication between an inside and an outside of the insertion empty space, the container accommodating the ejaculation promotion apparatus, and the decompression device includes: a negative pressure introducing section that communicates with an air vent hole provided in the container; a negative pressure generating section that generates a negative pressure to be introduced into the container through the negative pressure introducing section and the air vent hole; and a negative pressure adjusting section that adjusts the negative pressure supplied to the container through the negative pressure introducing section.

According to the present invention, the purpose of ejaculation can be efficiently achieved while the individual difference in the sensitivity of the user is flexibly dealt with by arbitrarily adjusting decompression of the pressure in the container that accommodates the core member to adjust the tightening force to the penis.

In the following the invention is further explained in detail by means of schematic examples disclosed in the figures which show:
- Figs. 1(a) and 1(b): a perspective view and an exploded perspective view illustrating a state in which a decompression device according to an embodiment of the present invention is mounted to an ejaculation promotion device through an adapter;
- Fig. 2: an exploded perspective view of an example of the ejaculation promotion device as an object to which the decompression device is applied;
- Fig. 3: an external perspective view of the decompression device (decompression device main body);
- Figs. 4(a) to 4(f): a front view, a left view, a right view, a rear view, a top view, and a bottom view, illustrating an external configuration of the decompression device main body;
- Figs. 5(a) and 5(b): a cross-sectional view along line A-A in Fig. 4(a) and a cross-sectional view along line B-B in Fig. 4(c);
- Figs. 6(a) and 6(b): a perspective view on a lever side and a perspective view on an opposite surface side, illustrating an internal configuration of the decompression device main body;
- Figs. 7(a) and 7(b): an upper-side perspective view on the lever side and a lower-side perspective view on the opposite surface side, illustrating the internal configuration of the decompression device main body;
- Figs. 8(a) and 8(b): diagrams illustrating a configuration and an attaching procedure of a battery box;
- Fig. 9: a schematic view for describing a principle of the decompression device of the present invention;
- Fig. 10: an external perspective view of an example of the adapter;
- Figs. 11(a) to 11(f): a front view, a left view, a right view, a rear view, a top view, and a bottom view of the adapter;
- Figs. 12(a) and 12(b): are sectional views of the ejaculation promotion device to which the adapter is mounted; and
- Fig. 13: a diagram illustrating a configuration example of a decompression device for an ejaculation promotion device according to a second embodiment of the present invention.

Hereinafter, the present invention will be described in detail according to embodiments illustrated in the drawings.

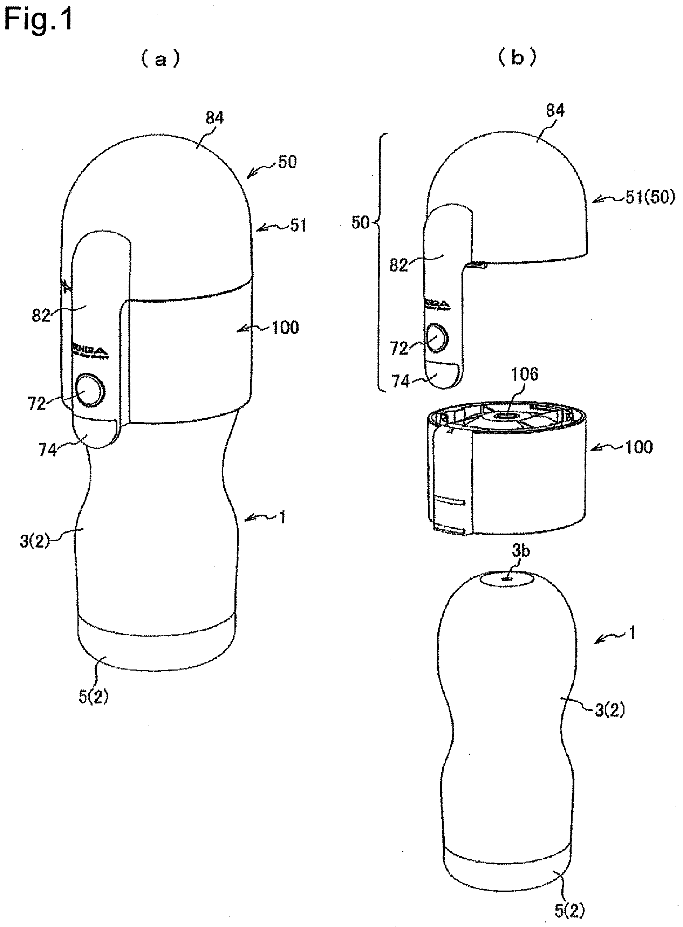

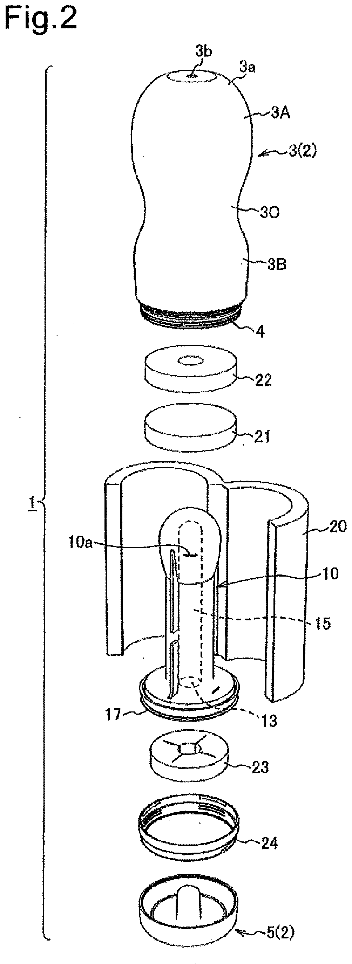

Figs. 1(a) and 1(b) are a perspective view and an exploded perspective view illustrating a state in which a decompression device according to an embodiment of the present invention is mounted to an ejaculation promotion device through an adapter, and Fig. 2 is an exploded perspective view of an example of the ejaculation promotion device as an object to which the decompression device is applied.

A decompression device 50 of the present invention illustrated in Figs. 1(a) and 1(b) is means that introduces, by being attached to the ejaculation promotion device through an adapter 100, a negative pressure into an inside of a container through an air vent hole provided in the container of the ejaculation promotion device to decompress the inside of the container.

First, an ejaculation promotion device 1 will be described on the basis of Fig. 2.

This ejaculation promotion device (sperm sampling device) 1 roughly includes a container 2 made of a tubular container body (container) 3 with one edge face in a longitudinal direction (axial direction) open, and a cap (container) 5 attached to or removed from an opening 4 of the container body 3 to open/close the opening 4, and a core member (ejaculation promotion apparatus) 10 made of a gel-like resin, accommodated in the container body 3, and including an insertion empty space 15 extending inward from an insertion hole 13 in the one edge face in the longitudinal direction (axial direction).

Further, a sheet 20 made of urethane foam provided between an outer periphery of the core member 10 and an inner wall of the container body 3 functions as buckling preventing means of the core member and as a filter that holds lubricating oil (lotion) not to spread to an outside when the lubricating oil inside the core member leaks through a cut (valve section) 10a cut and formed in the core member. A filter 21 made of urethane foam provided between a tip end of the core member and a tip end inner wall of the container body is means that holds the lubricating oil (lotion) leaking from an inside of the core member not to spread to an outside. A torus-shaped member 22 made of urethane provided to a tip end side of the filter 21 is means that cancels air-borne sound leaking from the inside of the core member.

Torus-shaped inner urethane 23 and a ring 24 made of a hard resin are provided between an insertion hole side (rear section side) of the core member and the cap 5. The torus-shaped inner urethane 23 may be provided to an inside on the insertion hole side of the core member. The inner urethane 23 is means that produces a soft feeling and supports smooth insertion when the penis is inserted through the insertion hole in the core member. The ring 24 is a member used when the core member 10 is inserted into and fit into the container body.

The container body 3 is configured from a resin material having a required thickness, and is a non-cylindrical body where an outer diameter of an intermediate portion (small-diameter portion) 3C is smaller than outer diameters of both end sections (large-diameter portions 3A and 3B) in the longitudinal direction. The shape of a connected portion of the large-diameter portion 3A and the intermediate portion 3C, and a contour of a connected portion of the large-diameter portion 3B and the intermediate portion 3C are arc shapes (curved shapes).

An air vent hole 3b as a small hole for air removal is formed in the other end section 3a of the container body (container) 3 as needed, and is sealed with a seal (not illustrated) at the time of non-use. At the time of use, the seal is removed, and the air vent hole 3b is closed and opened with a finger, so that the degree of adhesion or a feeling of adhesion (a feeling of absorption or a feeling of sucking) between an inner wall of the core member 10 and a penis can be adjusted. That is, tightening force becomes large because the penis adheres to the inner wall of the core member in a state where the air vent hole 3b is blocked, and the tightening force becomes small in a state where the air vent hole is opened. The tightening force (a pressure difference from the atmospheric pressure) is slightly changed by an operation to open/close the air vent hole, and a stimulus can be changed.

In more detail, when the penis is inserted into the insertion empty space 15 through the insertion hole 13 in the state where the air vent hole 3b is opened, the core member 10 can maintain original flexibility (deformable), and thus the penis can smoothly move back and forth inside the insertion empty space. Especially, the cut (valve section) 10a provided in a wall portion of the insertion empty space of the core member is opened when an internal pressure in the insertion empty space is increased, and is closed when the insertion empty space is decompressed. Therefore, the cut 10a is closed in a check valve manner when the penis is moved in a pulled out direction while allowing the smooth insertion when the penis is inserted. Therefore, the inside of the insertion empty space approaches a vacuum state, and an effect to cause the inner wall of the insertion empty space to absorb the penis moving in the pulled out direction, occurs.

Meanwhile, when the penis is inserted in the insertion empty space 15 in the state where the air vent hole is opened, and then the air vent hole 3b is blocked and the penis is moved toward the insertion hole 13 side, a phenomenon called vacuum occurs, where an inner wall of the insertion empty space is stuck to an outer surface of the penis with relatively strong force. In the vacuum state, the penis is stuck to the inner wall of the insertion empty space, and thus the penis less smoothly moves back and forth in the insertion empty space. However, a feeling of sticking can be obtained.

The core member (ejaculation promotion apparatus) 10 is an approximately bag-like body configured from a gel-like resin having viscosity such as elastomer or gel-like rubber. The core member 10 includes a large-diameter flange (annular rib) 17 in an insertion-side end section, and the insertion empty space 15 formed in communication with an inside of the small-diameter insertion hole 13. Projections, folds, or the like are formed in the insertion empty space 15 in an arbitrary arrangement. The slit-like cut 10a that allows the insertion empty space 15 and an outside to communicate is formed in an appropriate place of a side surface close to the tip end of the core member. The cut 10a is closed on a steady basis due to elasticity of the elastomer. However, the cut 10a is opened and functions as a check valve to discharge the air in the insertion empty space 15 into an outside when the internal pressure is increased. An appropriate amount of lotion or the like as lubricating oil is filled in the insertion empty space 15 in advance.

By the way, as described above, there are individual differences in strength of a stimulus that promotes ejaculation of a user, and a weak stimulus is sufficient for some and a strong stimulus is necessary for others to get a desired effect. To obtain the strong stimulus, an increase in adhesion force (absorption force) of the core member to the penis front end section (glans) is effective. However, there is a limit on the obtainable absorption force only by the operation to block the air vent hole 3b with a finger, and the purpose of ejaculation cannot be achieved by a person who needs the strong stimulus.

That is, to increase the stimulus to be added to the penis front end section when the penis rubs in the state of being inserted in the insertion empty space, it is effective to actively decompress the internal pressure in a space S (the internal pressure in the container body) formed between the core member and the container body to increase the adhesion force (absorption force) between the penis front end section and the core main body.

The present invention provides the decompression device 50 that can arbitrarily decrease (increase/decrease) the internal pressure in the container 2.

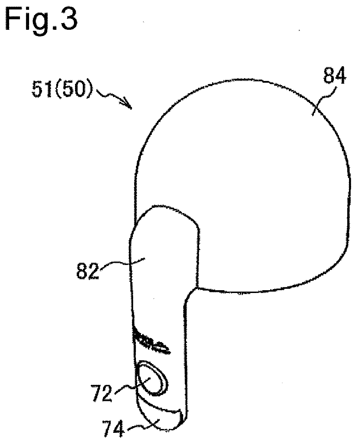

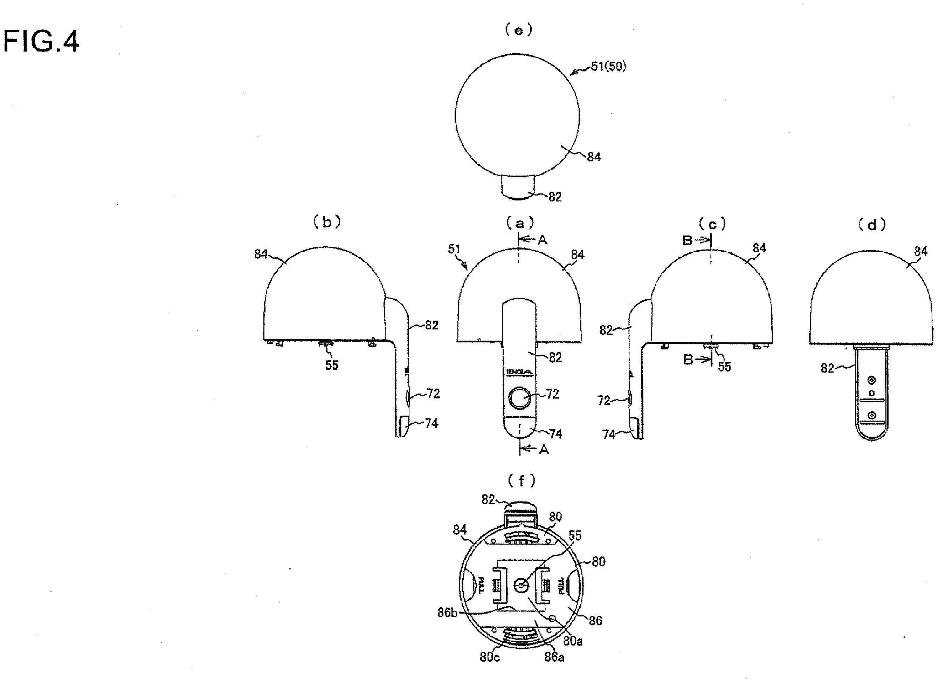

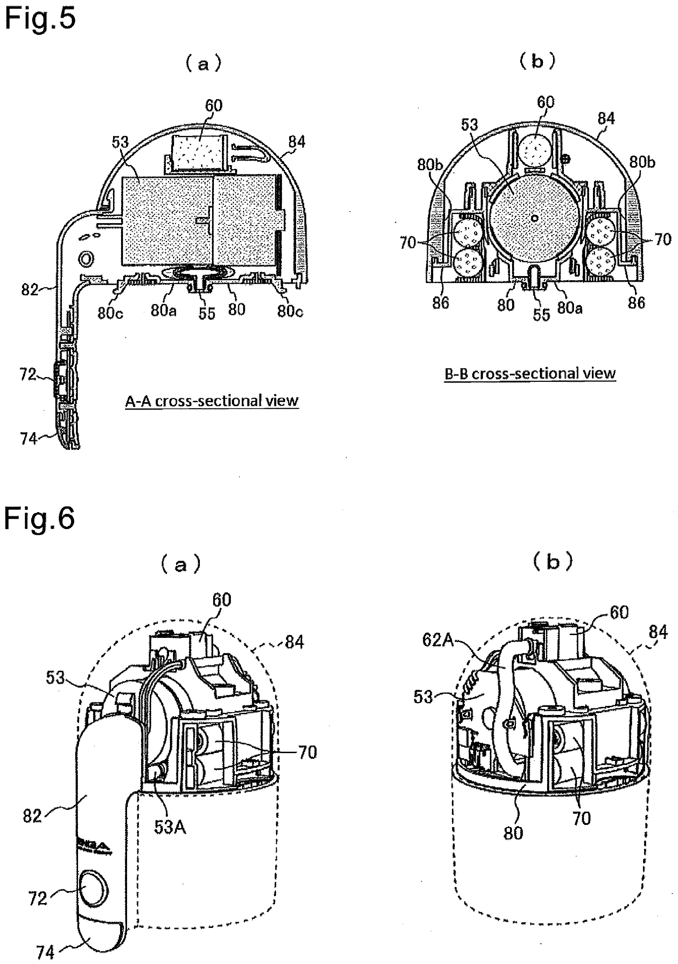

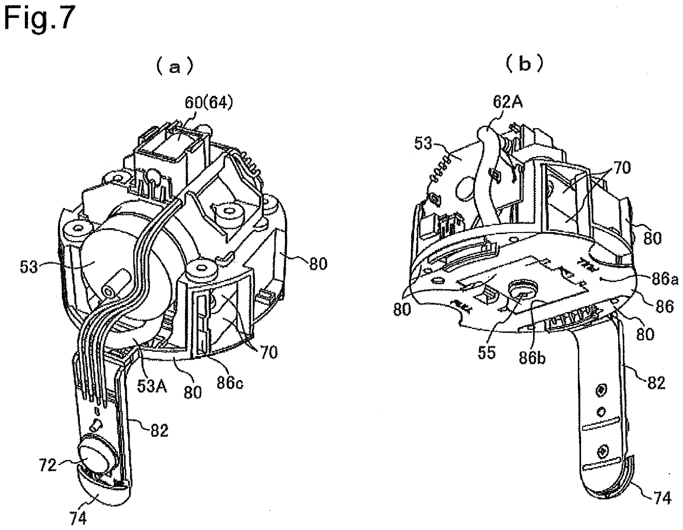

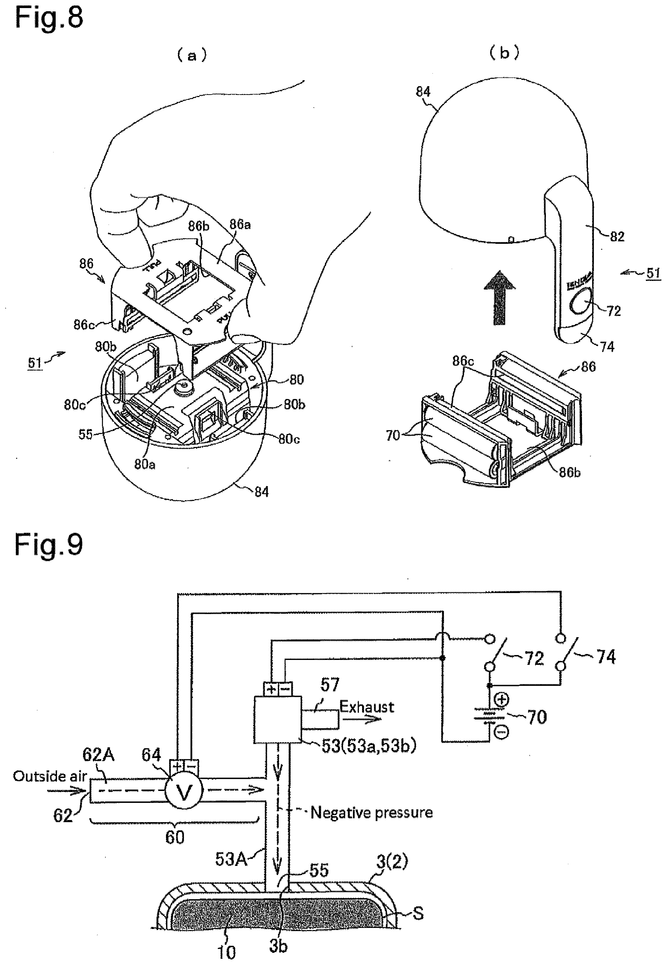

Fig. 3 is an external perspective view of the decompression device (decompression device main body), Figs. 4(a) to 4(f) are a front view, a left view, a right view, a rear view, a top view, and a bottom view, illustrating an external configuration of the decompression device main body, and Figs. 5(a) and 5(b) are a cross-sectional view along line A-A in Fig. 4(a) and a cross-sectional view along line B-B in Fig. 4(c). Figs. 6(a) and 6(b) are a perspective view on a lever side and a perspective view on an opposite surface side, illustrating an internal configuration of the decompression device main body, and Figs. 7(a) and 7(b) are an upper-side perspective view on the lever side and a lower-side perspective view on the opposite surface side, illustrating the internal configuration of the decompression device main body. Figs. 8(a) and 8(b) are diagrams illustrating a configuration and an attaching procedure of a battery box. Fig. 9 is a schematic view for describing a principle of the decompression device of the present invention.

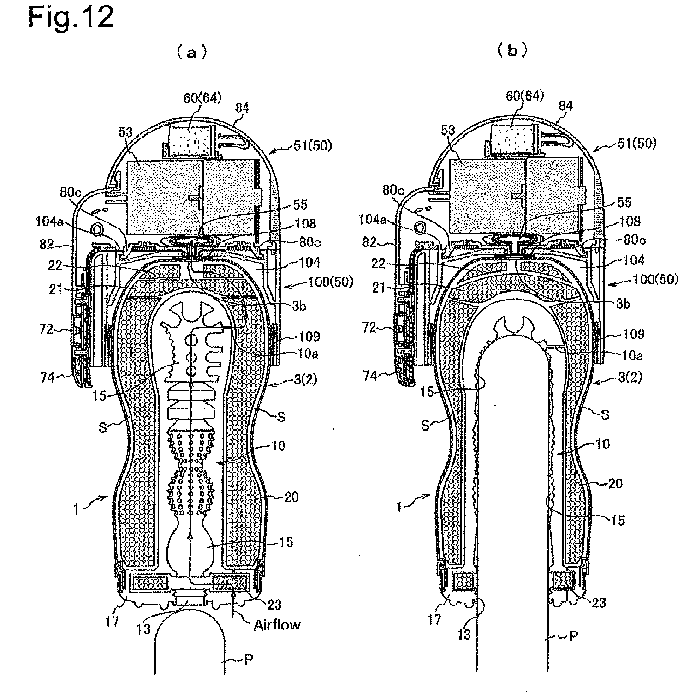

The decompression device 50 is means that decompresses the container body by being operated in a state of being mounted to an outer surface of the ejaculation promotion device 1 including the air vent hole 3b, and introducing a negative pressure into an inside of the container body. It is the space S (see Figs. 12(a) and 12(b)) that is decompressed by the decompression device, the space S being formed by the inner wall of the container body and the flange section 17 of the core member, which closes the opening of the container body. There are some types in which the opening of the container body is not closed by the flange section of the core member depending on the types of the ejaculation promotion device. Even in this case, an object to be decompressed is a space between an inner wall of a container body (container) and a core member.

The decompression device 50 roughly includes a decompression device main body 51, and an adapter 100 mounted to the decompression device main body 51 in an attachable and removable manner and directly connected to the ejaculation promotion device 1.

The decompression device main body 51 roughly includes a negative pressure generating section 53 that generates a negative pressure, a negative pressure introducing section 55 that decompresses the inside of the container body by introducing the negative pressure generated by the negative pressure generating section 53 into the air vent hole 3b provided in the container body 3, and a negative pressure adjusting section 60 that adjusts a value of the negative pressure to be supplied to the container body through the negative pressure introducing section 55. The negative pressure introduced into the container body is directly introduced into the space S between the inner wall of the container body and the outer surface of the core member outer surface, and is also introduced into the inside of the insertion empty space 15 through the cut 10a as a slit provided in the core member.

In the decompression device 50 according to the present example, the decompression device main body 51 is mounted to the container 2 through the adapter 100 instead of being directly mounted to the container (container body) of the ejaculation promotion device 1. However, the decompression device main body may be directly mounted to the container. In this case, the decompression device main body 51 serves as the decompression device 50. For such a configuration, a configuration to be air-tightly connected with the container body of the ejaculation promotion device may just be added to a bottom surface of the decompression device main body 51. For example, such a configuration can be realized by integrating the decompression device main body with the adapter 100.

Note that a plurality of types of the adapter 100 suitable for containers of ejaculation promotion devices having different shapes and sizes is provided, and is replaced and applied to one decompression device main body 51, thereby to be applicable to a wide variety of ejaculation promotion devices.

The decompression device main body 51 includes, as illustrated in the explanatory diagram of the principle of Fig. 9, a negative pressure generating section 53 made of a pump 53a (for example, diaphragm pump) and a motor 53b, a negative pressure introducing section (suction port) 55 that introduces the negative pressure generated by the negative pressure generating section 53 into the container body through the air vent hole 3b and decompresses, an exhaust section 57 that exhausts the air in the container body, the air having been suck through the negative pressure introducing section 55, a negative pressure adjusting section 60 connected between the negative pressure generating section 53 and the negative pressure introducing section 55 (another appropriate place), a battery 70 as a power source that supplies the power to the negative pressure generating section 53 and the negative pressure adjusting section 60, a power source switch 72 that turns ON/OFF the supply of the power to the negative pressure generating section, an operation switch (adjustment switch or air release button) 74 of the negative pressure adjusting section 60, and wiring electrically connecting the sections, the battery, and the switches.

Fig. 9 illustrates an example in which the negative pressure introducing section 55 is directly connected with the air vent hole 3b of the container. However, as described below, the negative pressure introducing section 55 may be connected with and communicate with the air vent hole 3b of the container through the adapter 100.

The negative pressure adjusting section 60 includes an outside air introducing section 62 (outside air introduction tube 62A) that introduces the outside air, and an air release electromagnetic valve (solenoid) 64 that supplies the outside air to between the negative pressure generating section and the negative pressure introducing section and disconnects the supply by being provided to the outside air introducing section 62 and opened/closed.

The electromagnetic valve 64 includes a solenoid main body (not illustrated) and a plunger supported by the solenoid main body in a freely movable forward and backward. The electromagnetic valve 64 is configured to open/close the outside air introduction tube 62A according to the position of the plunger and to finely adjust a releasing amount (closing amount).

In a case where the operation switch 74 of the electromagnetic valve is pressed in a state where the power source switch 72 is turned ON to operate the negative pressure generating section 53, the solenoid that configures the electromagnetic valve is continuously operated (release operated) to continuously mix the outside air to the negative pressure supplied to the container body through the negative pressure introducing section 55 during the time to press the operation switch. For example, during the period of pressing the operation switch 74, the outside air through the outside air introducing section 62 is mixed with the negative pressure from the negative pressure generating section 53, so that the value of the negative pressure introduced through the negative pressure introducing section can be decreased and the pressure in the container body can be gradually increased. Further, the negative pressure can be cancelled in a stepwise manner by finely pressing the operation switch 74 a plurality of times. In a case where the operation switch 74 is not depressed, the negative pressure generated by the negative pressure generating section 53 is supplied to the container body to the maximum.

Note that, by mounting control means (not illustrated), rhythmic program control to adjust ON/OFF of decompression, strength of the negative pressure, negative pressure supply timing, and the like become available, and adjustment of suction in rhythms optimum for individual users becomes available, leading to a possibility to a help to ED treatment.

A configuration example of the decompression device (main body) that realizes the principle illustrated in Fig. 9 will be described on the basis of the Figs. 4 to 8.

The decompression device main body 51 roughly includes a base member 80 that holds the negative pressure generating section 53, the negative pressure introducing section (suction port) 55, the exhaust section 57, the negative pressure adjusting section 60, and the like, a lever 82 protruding downward from an appropriate place on an outer periphery of the base member, a dome-like exterior case 84 with a lower surface open, attached to or removed from the base member 80, the lower surface covering the negative pressure generating section 53, the negative pressure introducing section 55, the exhaust section 57, the negative pressure adjusting section 60, and the like on the base member when mounted to the base member, and a battery box 86 (battery 70) attached to or removed from the lower surface of the base member 80.

The power source switch 72 of the negative pressure generating section and the operation switch 74 of the electromagnetic valve are mounted in the lever 82, and can be operated with a finger. As described later, the lever 82 protrudes in an outer diameter direction in an L-shaped manner from an outer peripheral surface of the exterior case 84 to cause the entire lever to overhang to an outside surface of the adapter when the adapter 100 is mounted to the lower surface of the decompression device main body.

A plurality of types of the adapters 100, each of which includes an attaching section 104 conforming to the ejaculation promotion devices 1 having different sizes, can be selectively attached to or removed from the lower surface of the base member 80. A section to be locked 80c to be attachably or removably locked with a locking section 104a provided in the attaching section 104 of the adapter is provided in the lower surface of the base member 80.

A negative pressure introduction tube 53A (Figs. 7(a) and 7(b)) extending from the negative pressure generating section 53 communicates with the negative pressure introducing section (suction port) 55 provided protruding to a central portion of a bottom surface of the base member 80. Therefore, when the negative pressure generating section 53 is operated, the negative pressure is introduced through the negative pressure introducing section 55. The negative pressure introducing section 55 can decompress the space S in the container body by communicating with the air vent hole 3b of the container through the adapter 100.

By introducing the outside air supplied to the negative pressure introduction tube 53A through the outside air introduction tube 62A that configures the negative pressure adjusting section 60, the value of the negative pressure to be supplied to the negative pressure introducing section 55 can be adjusted. In a case where no outside air is introduced, the negative pressure itself generated by the negative pressure generating section 53 is supplied to the negative pressure introducing section 55. In a case where the outside air is introduced, the negative pressure is decreased by the amount of the introduced outside air.

The battery box 86 includes a bottom plate 86a including an opening 86b for exposing a base member sections 80a including the negative pressure introducing section 55 when mounted to the bottom surface of the base member, and battery holding sections 86c respectively protruding from both end sections of the bottom plate toward the base member. When the battery holding sections 86c are fit into two groove sections 80b (Figs. 8(a) and 8(b)) provided in corresponding positions of the base member 80, the battery 70 held by the battery holding section 86c and a terminal (not illustrated) provided on the base member side become connected, so that the power supply becomes available to the negative pressure generating section 53 and the negative pressure adjusting section 60.

By use of the battery as a power source, reduction in size and weight becomes possible, and portability and operatability can be increased. The stimulus to the penis can be maintained to an appropriate value even if outputs of a motor and a pump driven by the battery are maximum. Therefore, there is no possibility of excessive suction force.

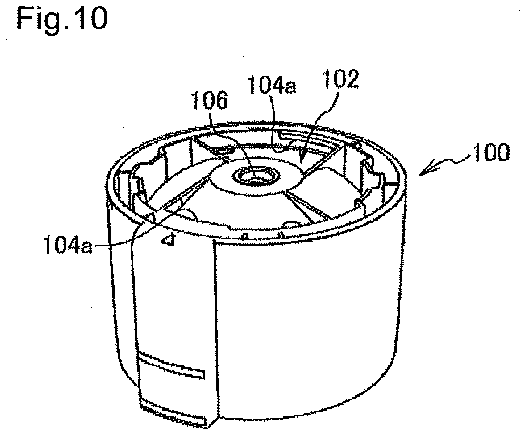

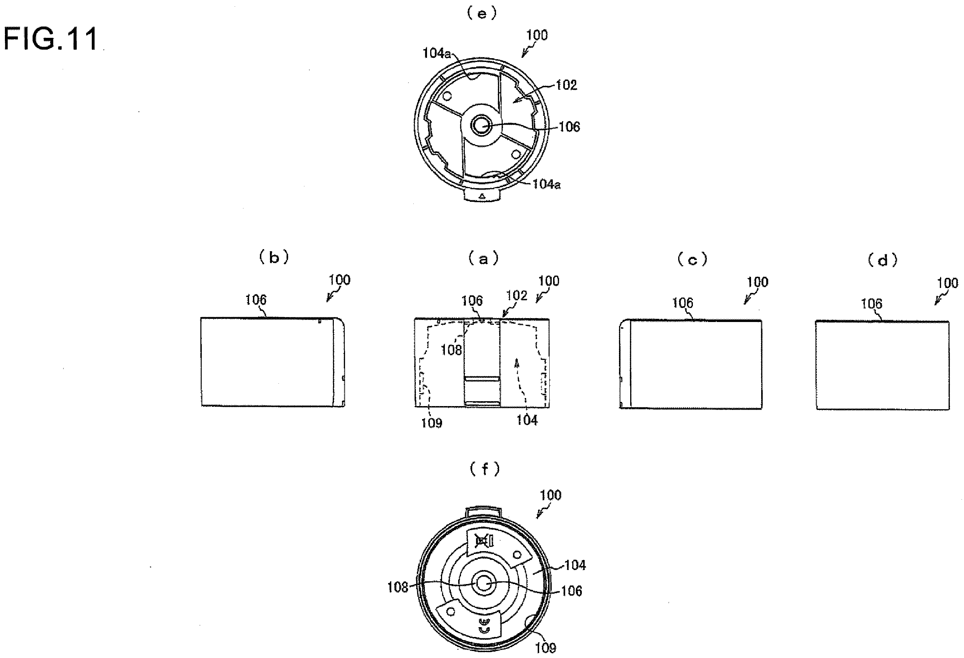

Fig. 10 is an external perspective view of an example of the adapter, and Figs. 11(a) to 11(f) are a front view, a left view, a right view, a rear view, a top view, and a bottom view of the adapter.

Further, Figs. 12(a) and 12(b) are sectional views of the ejaculation promotion device to which the adapter is mounted.

In the present example, the decompression device main body 51 is not directly mounted to the container body, and is connected to the container body through the adapter 100. That is, the adapter 100 includes a connecting section (upper surface) 102 attachably or removably connected to the lower surface of the decompression device main body 51, and the attaching section 104 including a communication connection hole 106 and causing the negative pressure introducing section 55 on the decompression device main body side to communicate with the air vent hole 3b when covering a part of the outer surface of the container body including the air vent hole 3b in a state of being appropriately connected with the decompression device main body in the connecting section 102.

The attaching section 104 as a recess formed in the lower surface of the adapter has a recessed surface shape that accords with the outer surface shape of the container body as an object to be mounted, and includes the negative pressure introducing section 55, and the communication connection hole 106 in the position corresponding to the air vent hole 3b. The communication connection hole 106 penetrates a ceiling surface of the adapter, communicates with the negative pressure introducing section 55 at an upper section, and communicated with the air vent hole 3b of the container body at a lower section.

The attaching section 104 is provided with the locking section 104a, and the section to be locked 80c provided in the lower surface of the base member 80 is attachably or removably locked with the locking section 104a. An attaching and removing structure of the decompression device main body to/from the adapter may be any form as long as the decompression device main body is not separated or dropped off during use of the ejaculation promotion device, and the attachment and removal is easy.

A ring-like depth section packing 108 provided to a lower peripheral edge of the communication connection hole 106, and a peripheral wall packing 109 provided to a while periphery of an inside wall of the attaching section 104 are provided to an inner wall of the attaching section 104. The depth section packing 108 is means that secures airtightness between the communication connection hole 106 and the air vent hole 3b, corresponding to a case in which the outer diameter or the contour on the ejaculation promotion device 1 (container body 3) has variation or some deformation. With this packing, a stable connection state and an introduction state of the negative pressure can be secured corresponding to the container bodies having various contours and shapes. The peripheral wall packing 109 can hold (prevent coming off of) the container and can secure airtightness only with the peripheral wall packing when the contour and the external diameter of the container body of the ejaculation promotion device adhere to the peripheral wall packing 109. In a case where the peripheral wall packing 109 cannot sufficiently secure the airtightness for some reasons such as a narrow diameter of the container body, and an unevenness in the peripheral surface, the depth section packing 108 can secure the definitive airtightness.

Note that both of the two packings 108 and 109 are not necessarily provided on a steady basis, and either one of them may be provided. Especially, sufficient airtightness can be secured in not only the case of providing both of the packings 108 and 109, but also the case of providing only the packing 109.

Further, the container may be directly attached to or removed from the decompression device main body itself by integrating the decompression device main body 51 and the adapter 100 (the attaching section 104 and the communication connection hole 106). In this case, the decompression device main body 51 serves as the decompression device 50, and the communication connection hole 106 serves as the negative pressure introducing section.

Next, a procedure of sampling sperm (promoting ejaculation) performed in a state where the container body of the ejaculation promotion device is decompressed will be described on the basis of Figs. 12(a) and 12(b).

Fig. 12 illustrates a state before a penis P is inserted. Since the negative pressure from the decompression device main body 51 (not illustrated) mounted to the upper side of the adapter 100 is introduced through the air vent hole 3b, and the internal pressure in the container 2 is decreased to a predetermined value or less, the cut 10a is opened, and the air flow as illustrated by the arrow in Fig. 12(a) is formed. In this state, the air in the insertion empty space 15 is discharged to an outside of the core member, and the insertion empty space is being vacuumed (decompressed). In this state, when the penis P is inserted into the insertion empty space 15, the penis is inserted while sucked by vacuum. When the penis is inserted into the insertion empty space, the air in the insertion empty space is discharged through the cut 10a, and the insertion empty space approaches the vacuum state.

The air in the container body and the insertion empty space is sucked and discharged to an outside, and the insertion empty space approaches the vacuum state as long as the decompression device main body 51 is continuously operated after the insertion of the penis illustrated in Fig. 12(b). A load to the penis is increased as the insertion empty space approaches the vacuum state. However, when the user operates the operation switch 74 of the electromagnetic valve that configures the negative pressure adjusting section 60, the vacuum state can be easily cancelled and the load to the penis can be decreased.

Note that the core member made of elastomer having stretchability, and urethane foam 20 having elasticity are provided inside the container body. Therefore, the penis can be stroked as long as the air slightly remains even if the insertion empty space approaches the vacuum state.

As described above, according to the decompression device 50 (decompression device main body 51) of the present invention, the internal pressure in the container 2 that accommodates the core member can be arbitrarily decreased (increased/decreased). The stimulus added to the penis front end section when the penis is inserted into the insertion empty space 15 and rubs in the state where the container body is decompressed can be sufficiently increased. This is because the adhesion force (absorption force) between the penis front end section and the core main body can be increased by decompressing proactively the internal pressure in the container 2.

In a case where the absorption force between the penis and the core member becomes excessive, the absorption force can be decreased by operating the negative pressure adjusting section 60, and the safety can be secured.

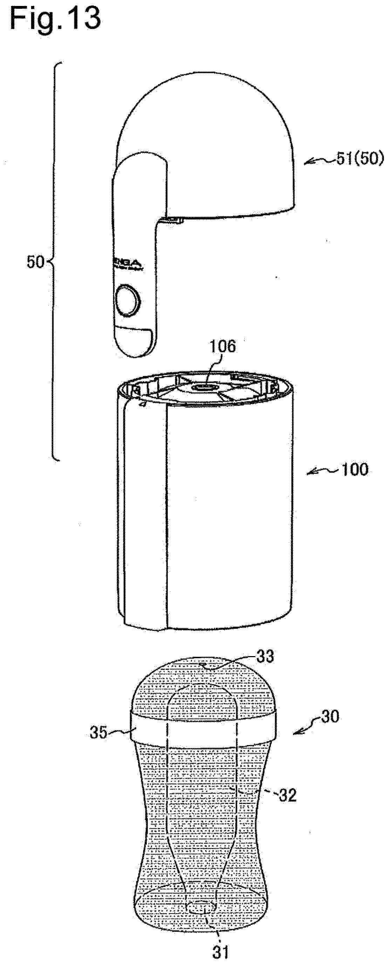

Next, Fig. 13 is a diagram illustrating a configuration example of a decompression device for an ejaculation promotion device according to a second embodiment of the present invention.

A decompression device 50 has a characteristic in that an object to be decompressed is a core member 30 itself, without a container.

In other words, the decompression device 50 is means that is attached to an ejaculation promotion apparatus 30 made of a gel-like resin, and including an insertion empty space 32 extending inward from an insertion hole 31 into which a penis is inserted and an air vent hole 33 that enables communication between an inside and an outside of the insertion empty space, and decompresses the insertion empty space. The decompression device 50 is characterized that it includes a negative pressure introducing section 55 communicating with the air vent hole 33, a negative pressure generating section 53 that generates a negative pressure to be introduced into the insertion empty space 32 through the negative pressure introducing section and the air vent hole 33, and a negative pressure adjusting section 60 that adjusts the negative pressure supplied to the insertion empty space 32 through the negative pressure introducing section.

The core member (ejaculation promotion device) 30 is made of a gel-like resin including the insertion empty space 32 extending upward from the insertion hole 31 in a lower edge face, and its use is sperm sampling, which is similar to the core member 10 according to the first embodiment. However, this core member 30 is a type of member gripped with a hand and directly used by a user without being accommodated in a container body.

In a case where the decompression device 50 is applied to the core member 30, an annular mounting part 35 made of hard plastic or the like is fixed to an appropriate place on an outer periphery of the core member 30, for example, and the mounting part 35 is configured to be locked by an attaching section 104 (not illustrated) provided to a lower surface of an adapter 100. When the air vent hole 33 provided in the core member 30 communicates with a communication connection hole 106 of the adapter when the mounting part 35 is held by the attaching section 104, the negative pressure introduced from the decompression device main body 51 is introduced into the core member 30, and the insertion empty space 32 is decompressed.

Note that the mounting part 35 is not essential, and the adapter can be directly mounted to the core member made of a gel-like resin by devising the shape of the core member and the configuration of the attaching section of the adapter.

Even in this embodiment, by operating the decompression device 50 in a battery system in the state where the penis is inserted in the insertion empty space, the insertion empty space is decompressed, and absorption force of an inner wall of the insertion empty space to the penis is increased and a stimulus is increased, whereby sperm sampling efficiency can be increased.

The decompression device for an ejaculation promotion device according to the first invention is means mounted to the ejaculation promotion device 1 including the ejaculation promotion apparatus 10 and the container 2, and which decompresses the container, the ejaculation promotion apparatus 10 being made of a gel-like resin, and including the insertion empty space extending inward from the insertion hole and the valve section 10a that enables communication between an inside and an outside of the insertion empty space, and the container 2 accommodating the ejaculation promotion apparatus, the decompression device including:
the negative pressure introducing section 55 that communicates with the air vent hole 3b provided in the container;
the negative pressure generating section 53 that generates the negative pressure to be introduced into the container through the negative pressure introducing section and the air vent hole; and
the negative pressure adjusting section 60 that adjusts the negative pressure supplied to the container from the negative pressure introducing section.

The negative pressure introducing section 55 may directly communicate with the air vent hole in the container, or may communicate with the air vent hole through another communication means (adapter 100). The negative pressure generated by the negative pressure generating section 53 may just be introducible into the container body. By decompression in the container body, the negative pressure is introduced into the insertion empty space through the valve section (cut) 10a in the ejaculation promotion apparatus 10, and the insertion empty space is decompressed.

By a conventional decompression method, sufficient decompression has been difficult because the penis needs to be moved backward in a taking-out direction while the air vent hole in the container is blocked (the container body is made to a closed space). In contrast, according to the decompression device of the present invention, strong suction force by electro-motion can be secured, and thus decompression (strong tightening) can be performed without moving the penis forward backward.

The negative pressure from the negative pressure generating section 53 decompresses the insertion empty space in the state where the penis is inserted in the insertion empty space, whereby the absorption force between the inner wall of the insertion empty space inner wall and the penis is increased, and the stimulus at the time of penis rubbing can be increased. The drive source of the decompression is a pump driven by a motor and is electrically driven, and thus a strong output can be obtained. Therefore, the purpose of ejaculation can be easily achieved by the user who needs a strong stimulus in the ejaculation.

That is, even if a person who needs a stronger stimulus than typical males in the ejaculation uses the ejaculation promotion device, an optimum, and necessary and sufficient stimulus (a feeling of vacuum and a feeling of sticking) is provided to the person, and the purpose of ejaculation (sperm sampling) can be achieved.

Further, when a tightening pressure becomes excessive, a decrease in operability, occurrence of an uncomfortable feeling, a decrease in the sensitivity of the user due to the excessive tightening pressure can be prevented by operating the negative pressure adjusting section 60.

A program can be made to realize ON/OFF of the power source and various operation modes in which the strength of the decompression is rhythmically. This can be a help to ED treatment.

The decompression device is a portable lightweight-type device using a battery as a power source, and is excellent in operatability.

By interposing a porous soft material such as urethane foam between the core member and the container, and between the core member and the air vent hole 3b, the lotion or the like leaking through the valve section of the core member can be absorbed and held by the urethane foam. Therefore, transfer of the lotion or the like to the decompression device side can be prevented. The urethane foam or the like that has absorbed the lotion can be reused by being washed.

In a case where the air vent hole exists in a top of the container, the lotion in the core member cannot directly leak at the time of suction if the valve section (normally elastically closed) is provided to a side section of the core member and the like avoiding the air vent hole. That is, by providing the valve section in a separated section avoiding the air vent hole of the container, the leakage of the lotion can be suppressed to the minimum. Even if the lotion leaks, the lotion can be sucked and held by filters made of urethane foam. Therefore, the lotion is not sucked by the decompression device side.

Even if the inside of the ejaculation promotion device is tried to be decompressed using a household vacuum cleaner, in place of the decompression device of the present invention, it is difficult to appropriately connect a suction port of the vacuum cleaner to the air vent hole of the ejaculation promotion device. Further, even if the suction port of the vacuum cleaner can be connected and the ejaculation promotion device can be decompressed, the suction force of the vacuum cleaner cannot be finely adjusted and becomes excessive for the penis even at minimum power, and is dangerous. Further, if the ejaculation promotion device is sucked by the vacuum cleaner, the core member made of a gel-like resin inside the container body may be sucked and damaged, and the filters (sponge) for lotion leakage prevention which are provided outside the core member may also be sucked.

Note that the outside air can be introduced into the container body and the internal pressure can be increased by operating only the negative pressure adjusting section 60 in the state where the negative pressure generating section 53 is stopped. In this case, a pump for outside air compression is provided to the negative pressure adjusting section.

By providing a rotation drive mechanism that rotates the container held on the decompression device side, the container (core member) can be rotated while being decompressed. When the core member is rotated with respect to the penis, a new and unconventional stimulus can be provided to the penis.

The decompression device for an ejaculation promotion device according to the second invention includes the attaching section 104 that allows the negative pressure introducing section 55 to communicate with the air vent hole when covering a part of the container body 3 including the air vent hole 3b.

By providing the negative pressure introducing section to the attaching section, the negative pressure introducing section and the air vent hole 3b may be directly connected, or the attaching section may be provided to an another member like the adapter 100.

The decompression device for ejaculation promotion device according to the third invention includes the decompression device main body 51 including the negative pressure introducing section 55, the negative pressure generating section 53, and the negative pressure adjusting section 60, and the adapter 100 attached to or removed from the decompression device main body, and the attaching section is provided to the adapter.

The air vent hole of the container may be directly connected to the negative pressure introducing section 55 of the decompression device main body 51, or the negative pressure introducing section 55 may be connected with the air vent hole through the attaching section 104 (communication connection hole 106) provided to the adapter. An advantage of providing the adapter 100 is that a plurality of types of the adapters is prepared corresponding to the ejaculation promotion devices (containers) having different contours and structures, and one decompression device main body can correspond to the different ejaculation promotion devices.

A connecting member having configurations like the decompression device main body and the adapter may be integrated.

The decompression device for an ejaculation promotion device according to the fourth invention is means mounted to the ejaculation promotion apparatus 30 including the insertion empty space and the air vent hole 33, and which decompresses the insertion empty space, the ejaculation promotion apparatus 30 being made of a gel-like resin, and including the insertion empty space extending inward from the insertion hole and the air vent hole 33 that enables communication between an inside and an outside of the insertion empty space, the decompression device including: the negative pressure introducing section that communicates with the air vent hole; a negative pressure generating section that generates a negative pressure to be introduced into the insertion empty space through the negative pressure introducing section and the air vent hole; and the negative pressure adjusting section that adjusts the negative pressure supplied to the insertion empty space through the negative pressure introducing section.

According to the present invention, the decompression device can be directly mounted to the ejaculation promotion apparatus made of a gel-like resin and can be used.

### LIST OF REFERENCES

- 1: ejaculation promotion device
- 2: container
- 3: container body
- 3A: large-diameter portion
- 3B: large-diameter portion
- 3C: intermediate portion
- 3a: other end section
- 3b: air vent hole
- 4: opening
- 5: cap
- 10: core member (ejaculation promotion apparatus)
- 10a: cut (valve section)
- 13: insertion hole
- 15: empty space
- 20: urethane foam
- 21: filter
- 23: inner urethane
- 24: ring
- 30: core member (ejaculation promotion apparatus)
- 31: insertion hole
- 32: empty space
- 33: air vent hole
- 35: mounting part
- 50: decompression device
- 51: decompression device main body
- 53: negative pressure generating section
- 53A: tube
- 53a: pump
- 53b: motor
- 55: negative pressure introducing section
- 57: exhaust section
- 60: negative pressure adjusting section
- 62: outside air introducing section
- 62A: outside air introduction tube
- 64: electromagnetic valve
- 70: battery
- 72: power source switch
- 74: operation switch
- 80: base member
- 80a: base member section
- 80b: groove section
- 82: lever
- 84: exterior case
- 86: battery box
- 86a: bottom plate
- 86b: opening
- 86c: battery holding section
- 100: adapter
- 102: connecting section
- 104: attaching section
- 106: connection hole
- 108: depth section packing
- 109: peripheral wall packing

## Claims

1. A decompression device (50) for an ejaculation promotion device (1), the decompression device (50) being configured to be mounted to the ejaculation promotion device (1) including an ejaculation promotion apparatus (10) and a container (2), and being configured to decompress the container (2),
the ejaculation promotion apparatus (10) being made of a gel-like resin and including:
an insertion empty space (15) extending inwardly from an insertion hole (13), and
a valve section (10a) that enables communication between an inside and an outside of the insertion empty space (15); and
the container (2) accommodating the ejaculation promotion apparatus (10),
the decompression device (50) comprising:
a negative pressure introducing section (55) that communicates with an air vent hole (33) provided in the container (2);
a negative pressure generating section (53) that generates a negative pressure to be introduced into the container (2) through the negative pressure introducing section (55) and the air vent hole (3b); and
a negative pressure adjusting section (60) that adjusts the negative pressure supplied to the container (2) through the negative pressure introducing section (55).

2. The decompression device (50) for an ejaculation promotion device (1) according to claim 1, comprising an attaching section (104) that allows the negative pressure introducing section (55) to communicate with the air vent hole (3b) when covering a part of the container (2) including the air vent hole (33).

3. The decompression device (50) for an ejaculation promotion device (1) according to claim 2, comprising:
a decompression device main body (51) including the negative pressure introducing section (55), the negative pressure generating section (53), and the negative pressure adjusting section (60); and
an adapter (100) being configured to be attached to or to be removed from the decompression device main body (51), wherein
the attaching section (104) is provided to the adapter (100).

4. A decompression device (50) for an ejaculation promotion device (1), the decompression device (50) being configured to be mounted to an ejaculation promotion apparatus (10) including an insertion empty space (15) and an air vent hole (10a), and being configured to decompress the insertion empty space (15),
the ejaculation promotion apparatus (10) being made of a gel-like resin;
the insertion empty space (15) extending inward from an insertion hole (13); and
the air vent hole (3b) enabling communication between an inside and an outside of the insertion empty space (15),
the decompression device (50) comprising:
a negative pressure introducing section (55) that communicates with the air vent hole (3b);
a negative pressure generating section (53) that generates a negative pressure to be introduced into the insertion empty space (15) through the negative pressure introducing section (55) and the air vent hole (3b); and
a negative pressure adjusting section (60) that adjusts the negative pressure supplied to the insertion empty space (15) through the negative pressure introducing section (55).
